# FASCICULE DE BREVET EUROPEEN

(11) **EP 0 708 103 B1**
(45) Date de publication et mention de la délivrance du brevet: **03.01.2001**
(21) Numéro de dépôt: 95402322.2
(22) Date de dépôt: 18.10.1995
(51) Int. Cl.: C07D 403/10, A61K 31/415

(54) **Procédé pour la préparation d'un dérivé de tétrazole sous deux formes cristallines et nouvelle forme cristalline de ce dérivé**
Verfahren zur Herstellung eines Tetrazolderivates in zwei kristallinen Formen und eine neue Kristallform dieses Derivates
Process for the preparation of a tetrazole derivative in two crystalline forms and a new crystalline form of this derivative

(30) Priorité: 19.10.1994 FR 9412459
(43) Date de publication de la demande: 24.04.1996
(73) Titulaire: Sanofi-Synthélabo, 75013 Paris (FR)
(72) Inventeur: Caron, Antoine, F-34560 Montbazin (FR); Chantreux, Dominique, F-34000 Montpellier (FR); Bouloumie, Colette, Résidence Le Golf-Bât. 5, F-34000 Montpellier (FR)
(74) Mandataire: Gillard, Marie-Louise

(56) Documents cités:
- EP-A- 0 420 237
- EP-A- 0 475 898
- JOURNAL OF MEDICINAL CHEMISTRY, vol.36, no.22, 29 Octobre 1993, WASHINGTON US pages 3371 - 3380 C.A. BERNHART ET AL. 'A New Series of Imidazolones: Highly Specific and Potent Nonpeptide AT1 Angiotensin II Receptor Antagonists'

## Description

La présente invention concerne un procédé pour la préparation de la 2-n.butyl-4-spirocyclopentane-1-[[2'-(tétrazol-5-yl)biphényl-4-yl]méthyl]-2-imidazoline-5-one sous deux formes cristallines différentes, une nouvelle forme cristalline de ce produit et des compositions pharmaceutiques contenant ladite nouvelle forme cristalline. Plus particulièrement, l'invention se rapporte à la préparation de la 2-n.butyl-4-spirocyclopentane-1-[[2'-(tétrazol-5-yl)biphényl-4-yl]méthyl]-2-imidazoline-5-one par réaction du nitrile correspondant avec un azoture alcalin et le chlorhydrate de triéthylamine et à l'isolement de ladite 2-n.butyl-4-spirocyclopentane-1-[[2'-(tétrazol-5-yl)biphényl-4-yl]méthyl]-2-imidazoline-5-one sous deux formes cristallines différentes.

La 2-n.butyl-4-spirocyclopentane-1-[[2'-(tétrazol-5-yl)biphényl-4-yl]méthyl]-2-imidazoline-5-one, ou 2-n.butyl-3-[[2'-(tétrazol-5-yl)biphényl-4-yl]méthyl]-1,3-diazaspiro[4.4]non-1-ène-4-one, est un puissant antagoniste des récepteurs de l'angiotensine II, préparé par réaction de la 2-n.butyl-1-[(2'-cyanobiphényl-4-yl)méthyl]-4-spirocyclopentane-2-imidazoline-5-one, ou 2-n.butyl-3-[(2'-cyanobiphényl-4-yl)méthyl]-1,3-diazaspiro[4.4]non-1-ène-4-one, soit avec l'azoture de tributylétain et le triphénylchlorométhane dans du xylène à reflux, par élimination du groupe protecteur triphénylméthyle et par isolement à partir d'une solution dans de l'acétate d'éthyle, dûment séchée (EP-A-0 454 511), soit directement avec l'azoture de tributylétain dans du xylène à reflux et isolement à partir d'une solution dans du dichlorométhane, dûment séchée (C.A. Bernhart et al., J. Med. Chem., 1993, 36, 3371-3380).

La préparation du 2-n.butyl-3-[[2'-(tétrazol-5-yl)biphényl-4-yl]méthyl]-1,3-diazaspiro[4.4]non-1-ène-4-one à partir 2-n.butyl-3-[(2'-cyanobiphényl-4-yl)méthyl]-1,3-diazaspiro[4.4]non-1-ène-4-one, par chauffage à reflux en présence d'azoture de tributylétain est également décrite dans EP 475 898.

Le composé ainsi préparé, auquel a été attribué la formule (A) 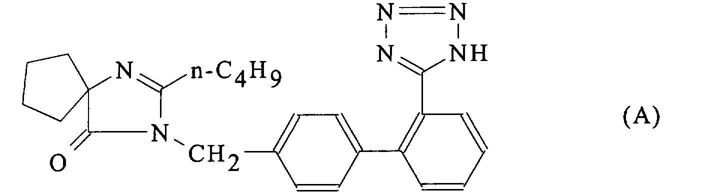 se présente sous forme d'aiguilles stables, non hygroscopiques qui peuvent être conservées et formulées sans aucune dégradation. Toutefois, il a été observé que la 2-n.butyl-3-[[2'-(tétrazol-5-yl)biphényl-4-yl]méthyl]-1,3-diazaspiro[4.4]non-1-ène-4-one obtenue selon les procédés décrits ci-dessus doit être formulée avec beaucoup d'attention car la poudre tend à rester attachée aux appareillages, par exemples aux tamis, aux poinçons ou aux parois des mélangeurs, à cause de son électrostaticité élevée.

Il est connu qu'un autre antagoniste non peptidique de l'angiotensine II, le losartan, à savoir le sel de potassium du 2-n.butyl-4-chloro-5-hydroxyméthyl-1-[2'-(tétrazol-5-yl)biphényl-4-yl]imidazole existe sous deux formes polymorphes dont l'une (la forme I) est régulièrement obtenue à la fin du procédé de préparation et l'autre (la forme II) est obtenue par chauffage à 250°C de la forme I. La forme I du losartan est stable à la température ambiante alors que la forme II l'est à température élevée. Par conséquent, la forme II se transforme graduellement dans la forme I, qui est thermodynamiquement plus stable à la température ambiante (K. Raghavan et al., Pharm. Res., 1993, 10, 900-904; L.S. Wu et al., Pharm. Res., 1993, 10, 1793-1795).

La transformation des nitriles en tétrazoles par réaction avec l'azoture de sodium et le chlorhydrate de triéthylamine est décrite en littérature.

Ainsi, par exemple, dans un article de P.R. Bernstein et E.P. Vacek, Synthesis, 1987, 1133-1134, les différentes méthodes de transformation du nitrile en tétrazole sont passées en revue et des conditions améliorées pour ladite transformation sont proposées. Notamment, dans cet article, il est indiqué que lorsque la réaction entre l'azoture de sodium et le chlorhydrate de triéthylamine est réalisée dans le diméthylformamide, on observe une formation "significative" de produit, accompagnée de produits de départ et de décomposition. Les conditions améliorées proposées par les auteurs consistent en l'utilisation de la 1-méthylpyrrolidin-2-one en tant que solvant à une température d'environ 150°C, à savoir à une température à laquelle on observe un reflux. Dans ces conditions, les rendements sont très variables selon les produits (de 60 à 98% avant et de 43 à 76% après cristallisation).

Dans le cas de la préparation du 5-{4-[2-(benzyloxycarboxamino)éthyl]phénoxyméthyl}-(1H)-tétrazole, décrite dans DE 3829431, le nitrile de départ est traité avec l'azoture de sodium et le chlorhydrate de triéthylamine dans la 1-méthylpyrrolidine-2-one à une température de 150°C pendant 8 heures.

Le document GB 2184121 décrit cinq préparations de tétrazoles antagonistes du leukotriène à partir des nitriles correspondants par réaction avec l'azoture de sodium et le chlorhydrate de triéthylamine dans du diméthylformamide à une température de 130-135°C à 160°C. Les rendements en produit final de ces préparations ne sont pas précisés, mais P.R. Bernstein et E.P. Vacek, dans l'article cité plus haut, ont montré que les rendements en tétrazole sont faibles lorsqu'on opère dans du diméthylformamide.

Par ailleurs, dans la série des dérivés du biphénylméthane, la transformation des dérivés cyano par chauffage en présence d'azoture de sodium et de chlorhydrate de triéthylamine a été décrite dans la demande de brevet EP 420 237.

Selon tous les enseignements des documents ci-dessus, ainsi que selon ceux de C. A. Bernhart et al. et de EP 0 454 511, cités plus haut, le produit final est isolé par évaporation du solvant et cristallisation éventuelle.

Il a été maintenant trouvé que si l'on cristallise la 2-n.butyl-3-[[2'-(tétrazol-5-yl)biphényl-4-yl]méthyl]-1,3-diazaspiro[4.4]non-1-ène-4-one dans un solvant plus ou moins pauvre en eau, on peut obtenir soit la 2-n.butyl-3-[[2'-(tétrazol-5-yl)biphényl-4-yl]méthyl]-1,3 -diazaspiro[4.4]non-1-ène-4-one sous la forme cristalline correspondant à celle du produit obtenu selon C.A. Bernhart et al. ou selon EP-A-0 454 511 cités ci-dessus, ci-après désignée "Forme A", soit la 2-n.butyl-3-[[2'-(tétrazol-5-yl)biphényl-4-yl]méthyl]-1,3-diazaspiro[4.4]non-1-ène-4-one sous une forme cristalline nouvelle, très stable, ayant une structure bien définie, ci-après désignée "Forme B". Plus particulièrement, il a été trouvé que la nouvelle forme cristalline de la 2-n.butyl-3-[[2'-(tétrazol-5-yl)biphényl-4-yl]méthyl]-1,3-diazaspiro-[4.4]non-1-ène-4-one (Forme B) est au moins aussi stable que la Forme A décrite dans EP-A-0 454 511 et J. Med. Chem., 1993, 36, 3371-3380, qu'elle ne se transforme pas spontanément dans la Forme A précédemment connue et qu'en plus, elle est beaucoup moins électrostatique que la Forme A et peut donc être plus aisement soumise à tout traitement dans les conditions usuelles de technique pharmaceutique. Par analyse diffractométrique aux rayons X du solide d'un monocristal, il a été constaté, de façon inattendue, que la nouvelle forme cristalline de la 2-n.butyl-3-[[2'-(tétrazol-5-yl)biphényl-4-yl]méthyl]-1,3-diazaspiro[4.4]non-1-ène-4-one est constituée par des cristaux tricliniques du tautomère pur ayant l'atome d'hydrogène du cycle tétrazole en position 2 et représentée par la formule (B)

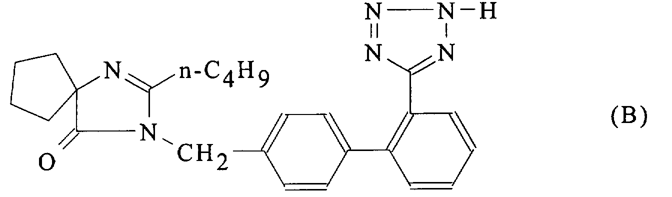

Enfin, il a été trouvé que la 2-n.butyl-3-[[2'-(tétrazol-5-yl)biphényl-4-yl]méthyl]-1,3-diazaspiro[4.4]non-1-ène-4-one peut être obtenue avec des rendements excellents, sans utiliser des dérivés d'étain, par réaction de la 2-n.butyl-3-[(2'-cyanobiphényl-4-yl]méthyl-1,3-diazaspiro[4.4]non-1-ène-4-one avec un azoture alcalin et le chlorhydrate de triéthylamine dans un solvant aprotique polaire inerte et par neutralisation en milieu aqueux d'un de ses sels alcalins, en l'isolant soit sous sa Forme A, soit sous sa Forme B. Notamment, les rendements en Forme A ou en Forme B, pures à au moins 99,8 %, sont de 80% ou plus.

La différence entre la nouvelle forme cristalline de la 2-n.butyl-3-[[2'-(tétrazol-5-yl)biphényl-4-yl]méthyl]-1,3-diazaspiro[4.4]non-1-ène-4-one selon la présente invention (Forme B) et la Forme A décrite par C.A. Bernhart et al., cités ci-dessus, ressort de l'examen des Figures 1 et 2, alors que les Figures 3 à 5 font état de la structure de la 2-n.butyl-3-[[2'-(tétrazol-5-yl)biphényl-4-yl]méthyl]-1,3-diazaspiro[4.4]non-1-ène-4-one dans les cristaux de la Forme B. Plus particulièrement:

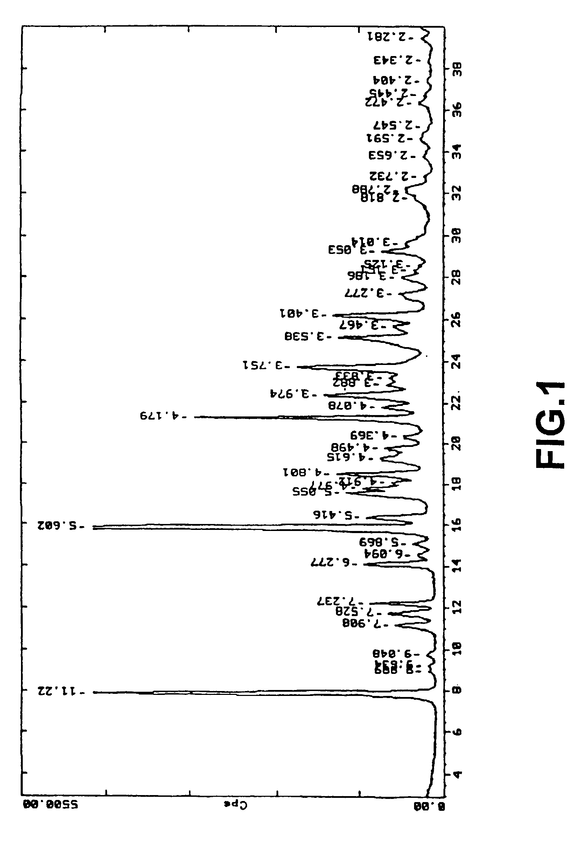

- la **Figure 1** donne le spectre de diffraction des rayons X de la poudre de la 2-n.butyl-3-[[2'-(tétrazol-5-yl)biphényl-4-yl]méthyl]-1,3-diazaspiro[4.4]non-1-ène-4-one, Forme B, qui met en évidence l'intensité maximale à la distance interréticulaire de 11,22 Å et de fortes intensités à 5,60 et 4,17 Å;

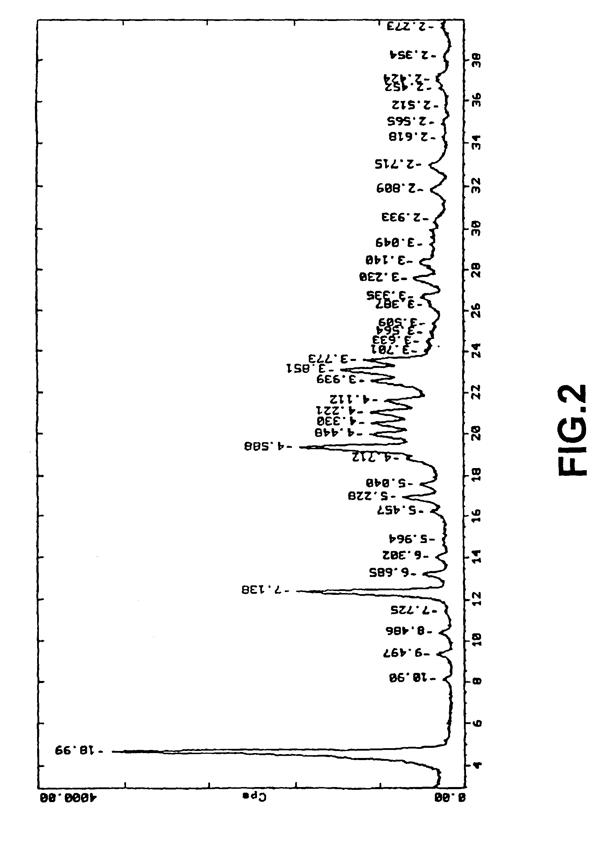

- la **Figure 2** donne le spectre de diffraction des rayons X de la poudre de la 2-n.butyl-3-[[2'-(tétrazol-5-yl)biphényl-4-yl]méthyl]-1,3-diazaspiro[4.4]non-1-ène-4-one, Forme A, qui met en évidence l'intensité maximale à la distance interréticulaire de 18,99 Å et des intensités relativement fortes à 7,13 et 4,58 Å;

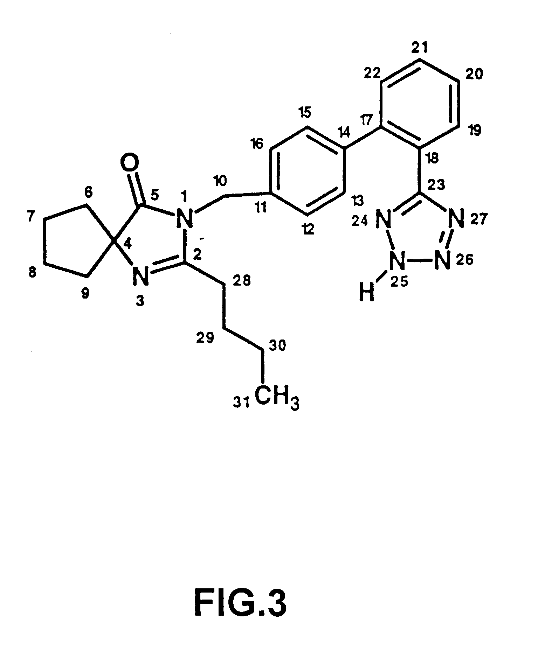

- la **Figure 3** donne la formule développée de la 2-n.butyl-3-[[2'-(tétrazol-5-yl)biphényl-4-yl]méthyl]-1,3-diazaspiro[4.4]non-1-ène-4-one, avec la numérotation des atomes, lorsqu'elle est présente dans la forme cristalline B;

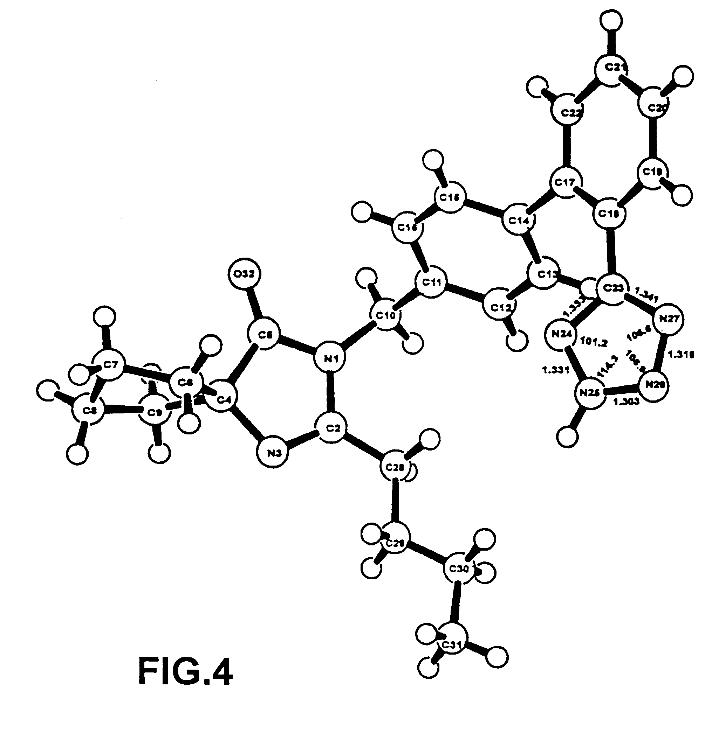

- la **Figure 4** donne la conformation spatiale de la molécule de la 2-n.butyl-3-[[2'-(tétrazol-5-yl)biphényl-4-yl]méthyl]-1,3-diazaspiro[4.4]non-1-ène-4-one, Forme B, dans le cristal;

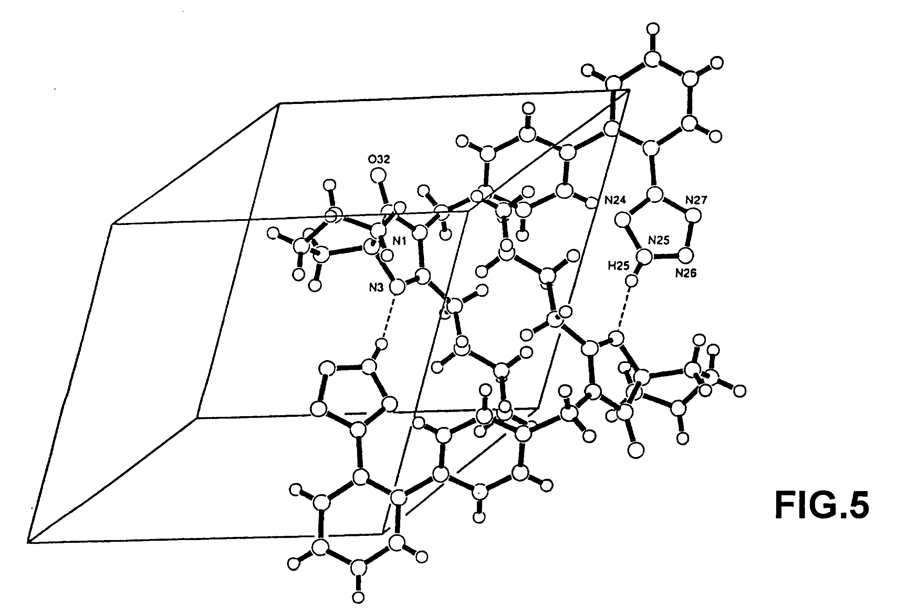

- la **Figure 5** montre le dimère cyclique de la 2-n.butyl-3-[[2'-(tétrazol-5-yl)biphényl-4-yl]méthyl]-1,3-diazaspiro[4.4]non-1-ène-4-one dans le cristal triclinique de la Forme B, formé par les liaisons hydrogène N(25)-H ... N(3); deux molécules de 2-n.butyl-3-[[2'-(tétrazol-5-yl)biphényl-4-yl]méthyl]-1,3-diazaspiro[4.4]non-1-ène-4-one sont disposées dans les cristaux de la Forme B pour former des "dimères" (bien qu'impropre car les deux molécules ne sont pas liées par des liaisons covalentes, on utilise le terme dimère parce que ces deux molécules sont assemblées par des liaisons hydrogène entre l'hydrogène en position 2 du tétrazole et l'atome d'azote en position 3 du cycle de l'imidazolinone, qui stabilisent la structure (2H)-5-tétrazolyle).

Ainsi, selon un de ses aspects, la présente invention a pour objet un procédé pour la préparation de la 2-n.butyl-3-[[2'-(tétrazol-5-yl)biphényl-4-yl]méthyl]-1,3-diazaspiro[4.4]non-1-ène-4-one caractérisé en ce que :
(a) on traite la 2-n.butyl-3-[(2'-cyanobiphényl-4-yl)méthyl]-1,3-diazaspiro[4.4]non-1-ène-4-one avec un azoture alcalin et le chlorhydrate de triéthylamine dans un solvant aprotique polaire inerte et on recupére la 2-n.butyl-3-[[2'-(tétrazol-5-yl)biphényl-4-yl]méthyl]-1,3-diazaspiro[4.4]non-1-ène-4-one ainsi obtenue sous forme d'un de ses sels alcalins en solution aqueuse;
(b) on neutralise en milieu aqueux le sel alcalin de la 2-n.butyl-3-[[2'-(tétrazol-5-yl)biphényl-4-yl]méthyl]-1,3-diazaspiro[4.4]non-1-ène-4-one ainsi obtenu jusqu'à un pH de 4,7 à 5,3; et
(c) on cristallise le produit ainsi précipité :
   - soit dans un solvant contenant moins d'environ 10% en volume d'eau pour isoler la 2-n.butyl-3-[[2'-(tétrazol-5-yl)biphényl-4-yl]méthyl]-1,3-diazaspiro[4.4]non-1-ène-4-one sous sa Forme A;
   - soit dans un solvant miscible à l'eau contenant plus d'environ 10% en volume d'eau pour isoler la 2-n.butyl-3-[[2'-(tétrazol-5-yl)biphényl-4-yl]méthyl]-1,3-diazaspiro[4.4]non-1-ène-4-one sous sa Forme B.

Plus particulièrement, dans l'étape (a), la 2-n.butyl-3-[(2'-cyanobiphényl-4-yl)méthyl]-1,3-diazaspiro[4.4]non-1-ène-4-one de départ est mélangée avec l'azoture alcalin, de préférence l'azoture de sodium et le chlorhydrate de triéthylamine dans un solvant aprotique polaire inerte tel que le N,N-diméthylformamide, le N,N-diméthylacétamide, le diméthylsulfoxyde, la 1-méthylpyrrolidin-2-one. Parmi les solvants aprotiques polaires utilisés dans cette étape, le diméthylformamide et la 1-méthylpyrrolidin-2-one sont particulièrement avantageux.

Bien que la réaction puisse être conduite au reflux, il a été constaté que pour l'économie du procédé l'utilisation de températures inférieures est très avantageuse car elle évite que, pendant le reflux, des composés azothydriques soient entrainés dans les réfrigérants, ce qui comporterait des risques pour la sécurité.

De préférence, le mélange réactionnel est chauffé à une température inférieure à la température de reflux, avantageusement de 10-30°C inférieure, notamment à une température de 110 à 140°C si, comme solvant, on utilise le diméthylformamide ou la 1-méthylpyrrolidin-2-one. Dans le diméthylformamide on opère en général à 115-125°C et dans la 1-méthylpyrrolidin-2-one la température optimale de réaction est de 120-130°C, même si on peut monter jusqu'à 140°C.

Selon la présente invention, on utilise de préférence des quantités équimoléculaires d'azoture de sodium et de chlorhydrate de triéthylamine dans des proportions de 1,5 à 5 moles par mole de nitrile de départ, avantageusement d'environ 2 à environ 4 moles par mole de nitrile. Dans ces conditions, on peut opérer en milieu concentré, même très concentré, en utilisant de 0,6 à 7 litres de solvant, par mole de nitrile de départ.

Après 6-20 heures de chauffage, la réaction est terminée et le mélange réactionnel est traité selon les techniques classiques. Notamment, le mélange est neutralisé par addition d'une base, par exemple un hydroxyde alcalin, en solution aqueuse, la phase aqueuse contenant les sels, notamment des chlorures et des azotures est écartée. La phase organique est alors traitée avec de l'eau et des solvants organiques tels que le toluène, l'acétate d'éthyle, éventuellement avec deux solvants différents de façon séquentielle, permettant d'éliminer les sous-produits de réaction.

La phase aqueuse ainsi isolée, contenant le sel alcalin de la 2-n.butyl-3-[[2'-(tétrazol-5-yl)biphényl-4-yl]méthyl]-1,3-diazaspiro[4.4]non-1-ène-4-one, est soumise à l'étape (b) qui consiste en une acidification, de préférence par addition d'acide chlorhydrique jusqu'à un pH de 4,7 à 5,3, de préférence de 4,8 - 5,2, de façon à obtenir une 2-n.butyl-3-[[2'-(tétrazol-5-yl)biphényl-4-yl]méthyl]-1,3-diazaspiro[4.4]non-1-ène-4-one brute ayant déjà un degré de pureté satisfaisant après avoir bien éliminé le solvant du produit.

Le rendement global à partir du nitrile est très élevé (jusqu'à 80-90% de la théorie).

Dans l'étape (c), le produit ainsi obtenu est soumis à une cristallisation afin d'obtenir la 2-n.butyl-3-[[2'-(tétrazol-5-yl)biphényl-4-yl]méthyl]-1,3-diazaspiro[4.4]non-1-ène-4-one soit sous sa Forme A, soit sous sa Forme B.

Pour obtenir la Forme A, le produit obtenu est cristallisé dans un solvant contenant moins d'environ 10% d'eau en volume, de préférence dans un solvant anhydre, la présence de ces quantités d'eau n'étant pas critique. Les solvants préférés pour cette opération sont les alcools, notamment l'éthanol 95% ou l'isopropanol, les conditions de cristallisation étant celles habituellement employées pour ce type d'opération.

Pour obtenir la Forme B, le produit obtenu à la fin de l'étape (b) est cristallisé sous agitation dans un solvant miscible à l'eau contenant une quantité d'eau supérieure à environ 10%. Dans cette cristallisation la présence de l'eau est critique. Les solvants organiques miscibles à l'eau qui peuvent être utilisés sont par exemple les alcools, notamment le méthanol, l'éthanol et l'isopropanol, les cétones, notamment l'acétone, les éthers comme le tétrahydrofurane ou le dioxane, les nitriles, notamment l'acétonitrile. Pour l'obtention de la Forme B, la technique est aussi celle habituellement utilisée pour les opérations de cristallisation.

La formation des Formes A et B est relativement indépendante de la vitesse de refroidissement et l'amorçage peut être utile, mais n'est pas indispensable.

Le pourcentage d'eau en volume dans le solvant organique a été fixé à environ 10% comme maximal et minimal pour l'obtention de la Forme A et de la Forme B, respectivement, mais ce pourcentage constitue une valeur limite qui dépend aussi du solvant organique utilisé. Par exemple, lorsqu'on utilise un mélange acétone/eau pour la préparation de la Forme B, une quantité de 9-11% d'eau est suffisante pour l'obtention de 100% de forme B. Il est cependant avantageux d'utiliser un pourcentage d'eau en volume d'au moins 15%, de préférence de 15 à 50%, pour la préparation de la Forme B. De même, lorsqu'on utilise par exemple l'isopropanol pour la préparation de la Forme A, celle-ci est obtenue même lorsque le solvant organique contient 9-11% d'eau. Notamment, il est préférable d'utiliser un solvant anhydre, bien que dans l'éthanol 95 on obtienne 100% de Forme A.

Le fait que la présence de l'eau et son absence soient critiques comporte la possibilité de passer d'une forme cristalline à l'autre par une recristallisation dans les conditions ci-dessus. En effet, il a été constaté que la Forme A peut être transformée dans la Forme B par recristallisation par exemple dans une solution hydroalcoolique, alors qu'une recristallisation dans, par exemple, de l'isopropanol donne encore la Forme A, même après amorçage avec la Forme B. De façon analogue, la Forme B se transforme en Forme A par recristallisation dans l'isopropanol.

Une telle observation a permis de mettre au point un procédé pour la préparation de la Forme B de la 2-n.butyl-3-[[2'-(tétrazol-5-yl)biphényl-4-yl]méthyl]-1,3-diazaspiro[4.4]non-1-ène-4-one à partir de la Forme A obtenue par les procédés connus, décrits par exemple dans l'article de C.A. Bernhart et al. et dans EP-A-0 454 511 ou bien à partir des sels alcalins de la 2-n.butyl-3-[[2'-(tétrazol-5-yl)biphényl-4-yl]méthyl]-1,3-diazaspiro[4.4]non-1-ène-4-one, notamment du sel de potassium.

Ainsi, selon un autre de ses aspects, la présente invention concerne un procédé pour la préparation de la Forme B de la 2-n.butyl-3-[[2'-(tétrazol-5-yl)biphényl-4-yl]méthyl]-1,3-diazaspiro[4.4]non-1-ène-4-one caractérisé en ce qu'on recristallise une 2-n.butyl-3-[[2'-(tétrazol-5-yl)biphényl-4-yl]méthyl]-1,3-diazaspiro[4.4]non-1-ène-4-one sous forme de produit brut ou sous la Forme A dans un solvant miscible à l'eau contenant au moins environ 10% d'eau.

Plus particulièrement, lorsque la 2-n.butyl-3-[[2'-(tétrazol-5-yl)biphényl-4-yl]méthyl]-1,3-diazaspiro[4.4]non-1-ène-4-one de départ est sous forme de produit brut, la cristallisation est généralement conduite dans les conditions illustrées ci-dessus, à savoir en utilisant un solvant comme un alcool, l'acétone, le tétrahydrofurane ou l'acétonitrile en présence d'au moins 10% d'eau, avantageusement d'au moins 15%, de préférence 15-50% d'eau. La 2-n.butyl-3-[[2'-(tétrazol-5-yl)biphényl-4-yl]méthyl]-1,3-diazaspiro[4.4]non-1-ène-4-one sous forme de produit brut utilisée comme composé de départ peut être celle obtenue à partir d'un sel alcalin de la 2-n.butyl-3-[[2'-(tétrazol-5-yl)biphényl-4-yl]méthyl]-1,3-diazaspiro[4.4]non-1-ène-4-one, après dissolution dans de l'eau, neutralisation avec un acide jusqu'à un pH de 4,7 à 5,3 et filtration du précipité. Cette procédure est conseillée lorsque la 2-n.butyl-3-[[2'-(tétrazol-5-yl)biphényl-4-yl]méthyl]-1,3-diazaspiro[4.4]non-1-ène-4-one est stockée sous forme d'un sel alcalin, de préférence de sel de potassium.

Lorsque la 2-n.butyl-3-[[2'-(tétrazol-5-yl)biphényl-4-yl]méthyl]-1,3-diazaspiro[4.4]non-1-ène-4-one de départ est sous sa Forme A, les conditions de cristallisation sont très flexibles car la quantité d'eau présente, toujours au moins 10%, peut varier de 10% à 100%. Cela est dû au fait que la Forme A de départ est déjà très pure et que, par conséquent, le solvant organique n'est plus nécessaire. Il faut néanmoins considérer que lorsqu'on opère dans l'eau seule, la transformation est très lente, et doit être accélerée par addition d'un acide tel que l'acide chlorhydrique jusqu'à un pH de 2-3, à savoir sans provoquer la formation du sel d'addition acide, tel que le chlorhydrate.

La nouvelle Forme B est isolée par simple filtration et dessication.

Bien que la présence d'eau soit critique pour la formation de la Forme B de la 2-n.butyl-3-[[2'-(tétrazol-5-yl)biphényl-4-yl]méthyl]-1,3-diazaspiro[4.4]non-1-ène-4-one, dans les conditions normales de formulation pharmaceutique on n'observe aucune transformation de la Forme A dans la Forme B. Plus particulièrement, par exemple, pendant la granulation par voie humide de la Forme A, on n'observe pas de formation de la Forme B.

Ainsi, selon un autre de ses aspects, la présente invention a pour objet la Forme B de la 2-n.butyl-3-[[2'-(tétrazol-5-yl)biphényl-4-yl]méthyl]-1,3-diazaspiro[4.4]-non-1-ène-4-one, caractérisée par le profil de diffraction des rayons X de la poudre donné dans le Tableau I.

Plus particulièrement, la Forme B de la 2-n.butyl-3-[[2'-(tétrazol-5-yl)biphényl-4-yl]méthyl]-1,3-diazaspiro[4.4]non-1-ène-4-one est aussi caractérisée par un point de fusion, déterminé par analyse calorimétrique différentielle (DSC), de 185-186°C et par des absorptions caractéristiques dans l'infrarouge à 1537, 1200 et 745 cm⁻¹.

Les propriétés physiques et le comportement de la nouvelle forme cristalline (Forme B) de la 2-n.butyl-3-[[2'-(tétrazol-5-yl)biphényl-4-yl]méthyl]-1,3-diazaspiro[4.4]non-1-ène-4-one selon la présente invention sont complètement différentes de ceux de la Forme A décrite par C.A. Bernhart et al. et EP 0 454 511 cités ci-dessus, comme il a été demontré en examinant les deux formes selon les méthodes et les techniques classiques.

Le profil de diffraction des rayons X de la poudre (angle de diffraction) a été établi avec un diffractomètre Siemens D500TT. Les spectres caractéristiques entre 5 et 40° (2 théta) sont présentés sur la Figure 1 pour la Forme B et sur la Figure 2 pour la Forme A. Les raies significatives de la Figure 1 sont consignées dans le Tableau I, alors que celles de la Figure 2 sont rassemblées dans le Tableau II.

Dans les Tableaux I et II, d est la distance interréticulaire et I/I₀ représente l'intensité relative, exprimée en pourcentage de la raie la plus intense.

**TABLEAU I**

| **d** | **I/I**_{**0**} |
|---|---|
| 11,22 | 100,00 |
| 7,90 | 12,02 |
| 7,52 | 13,79 |
| 7,23 | 18,60 |
| 6,27 | 20,14 |
| 6,09 | 6,47 |
| 5,86 | 7,42 |
| 5,60 | 98,76 |
| 5,41 | 19,45 |
| 5,05 | 24,67 |
| 4,97 | 20,36 |
| 4,91 | 12,92 |
| 4,80 | 27,33 |
| 4,61 | 15,90 |
| 4,49 | 14,73 |
| 4,36 | 9,86 |
| 4,17 | 62,84 |
| 4,07 | 15,39 |
| 3,97 | 30,34 |
| 3,88 | 14,32 |
| 3,83 | 13,56 |
| 3,75 | 37,28 |
| 3,53 | 26,48 |
| 3,46 | 12,42 |
| 3,40 | 27,88 |
| 3,27 | 11,03 |
| 3,18 | 10,42 |
| 3,15 | 7,28 |
| 3,12 | 6,11 |
| 3,05 | 15,50 |
| 3,01 | 9,49 |
| 2,81 | 7,11 |
| 2,78 | 9,40 |

**TABLEAU II**

| **d** | **I/I**_{**0**} |
|---|---|
| 18,98 | 100,00 |
| 10,89 | 5,81 |
| 9,49 | 7,43 |
| 8,48 | 6,60 |
| 7,13 | 46,23 |
| 6,68 | 11.25 |
| 6,30 | 7,45 |
| 5,45 | 8,85 |
| 5,22 | 16,82 |
| 5,03 | 11,81 |
| 4,71 | 15,91 |
| 4,58 | 45,40 |
| 4,44 | 26,12 |
| 4,32 | 25,44 |
| 4,22 | 25,86 |
| 4,11 | 21,72 |
| 3,93 | 25,46 |
| 3,85 | 33,89 |
| 3,77 | 27,76 |
| 3,38 | 9,09 |
| 3,33 | 11,75 |
| 3,23 | 13,68 |
| 3,14 | 11,99 |
| 2,80 | 8,97 |
| 2,71 | 9,50 |

L'analyse calorimétrique différentielle (DSC) des Formes A et B a été réalisée comparativement en utilisant un appareil DSC7 Perkin Elmer, calibré par référence à l'indium et au cyclohexane.

Pour l'analyse calorimétrique on a utilisé de 3 à 6 mg de la Forme A ou de 3 à 6 mg de la Forme B, telle qu'obtenue à l'exemple 2, dans une coupelle en aluminium sertie et percée, dans une zone de température de 20 à 200°C à une vitesse de chauffage de 10°C/minute. Le point de fusion et l'enthalpie de fusion sont indiqués dans le Tableau III. Le point de fusion correspond à la température caractéristique de la fusion obtenue par DSC. Cette valeur peut être définie également comme étant la température correspondant à l'intersection entre la ligne de base et la tangente à la montée du pic de fusions observées par DSC.

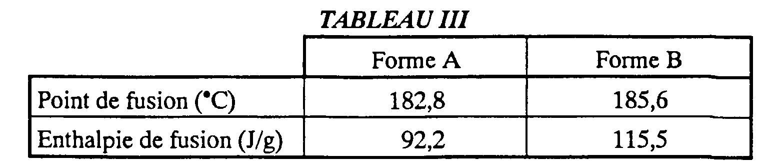

De ce tableau il ressort que la Forme B est thermodynamiquement plus stable que la Forme A.

La différence entre la nouvelle Forme B et la Forme A connue de la 2-n.butyl-3-[[2'-(tétrazol-5-yl)biphényl-4-yl]méthyl]-1,3-diazaspiro[4.4]non-1-ène-4-one a été également mise en évidence par spectroscopie infrarouge. Les spectres IR à Transformée de Fourier (FTIR) de 4000 cm⁻¹ à 600 cm⁻¹ ont été obtenus avec un spectromètre Nicolet 5PC de résolution 4 cm⁻¹. Les échantillons ont été préparés en mélangeant 2 mg de la Forme A ou de la Forme B et 200 mg de KBr, le tout comprimé sous 2 tonnes pendant 2 minutes. Chaque échantillon à été examiné après 32 accumulations.

La comparaison de raies caractéristiques, en termes de longueur d'onde (en cm⁻¹) et d'intensité (en pourcentage de transmittance) est illustrée dans le Tableau IV.

**TABLEAU IV**

| Longueur d'onde (cm-1) | % de transmittance | |
|---|---|---|
| | Forme A | Forme B |
| 745 | ∗ | 2,5 |
| 758 | 3,7 | ∗ |
| 781 | 17,8 | ∗ |
| 959 | 22,7 | ∗ |
| 1007 | 26,6 | 6,6 |
| 1177 | ∗ | 7,2 |
| 1179 | 23,5 | ∗ |
| 1200 | ∗ | 18,0 |
| 1238 | 26,1 | ∗ |
| 1383 | 20,9 | ∗ |
| 1537 | ∗ | 14,1 |

Il ressort du Tableau IV que la Forme B présente des absorptions caractéristiques à 745, 1200 et 1537 cm⁻¹ qui sont absentes dans la Forme A.

La structure particulière 2H-tétrazol-5-yle de la Forme B a été mise en évidence par la diffraction des rayons X d'un monocristal en utilisant un diffractomètre MSC-Rigaka AFC6S et les logiciles SHELXS-90 et SHELXS-93 sur une station de travail SG IRIS Indigo. La position des hydrogènes C-H a été générée à une distance de 0,95Å.

Les données cristallographiques, notamment les distances interplanaires (a, b, c) les angles (α, β, γ) et le volume de chaque cellule unitaire, sont indiquées dans le Tableau V

**TABLEAU V**

| **Données cristallographiques et établissement de la structure de la Forme B** | |
|---|---|
| Système cristallin Groupe spatial | triclinique P-1 |
| Dimensions de la cellule unitaire: | |
| a | 11,170(5) Å |
| b | 12,181(4) Å |
| c | 9,366(4) Å |
| α | 90,75(4) degrés |
| β | 105,24(4) degrés |
| γ | 112,92(3) degrés |
| volume | 1122,9(8) Å³ |

Les coordonnées atomiques de la Forme B de la 2-n.butyl-3-[[2'-(tétrazol-5-yl)biphényl-4-yl]méthyl]-1,3-diazaspiro[4.4]non-1-ène-4-one sont données dans le Tableau VI, la longueur des liaisons dans le Tableau VII, les angles entre les liaisons dans le Tableau VIII et les angles de torsion caractéristiques dans le Tableau IX.

**TABLEAU VI**

| **Paramètres de position de la Forme B** | | | |
|---|---|---|---|
| atome | x | y | z |
| N(1) | 0,4011(2) | 0,6617(2) | -0,2435(2) |
| C(2) | 0,3178(3) | 0,5684(3) | -0,3565(3) |
| N(3) | 0,3387(2) | 0,5866(2) | -0,4838(3) |
| C(4) | 0,4491(3) | 0,7065(2) | -0,4659(3) |
| C(5) | 0,4859(3) | 0,7514(2) | -0,3022(3) |
| C(6) | 0,4051(4) | 0,7912(4) | -0,5671(4) |
| C(7) | 0,5268(5) | 0,8643(4) | -0,6164(6) |
| C(8) | 0,5872(5) | 0,7781(5) | -0,6362(5) |
| C(9) | 0,5724(3) | 0,7036(3) | -0,5096(4) |
| C(10) | 0,4094(3) | 0,6654(3) | -0,0844(3) |
| C(11) | 0,3135(2) | 0,7106(2) | -0,0433(3) |
| C(12) | 0,2025(3) | 0,6349(2) | -0,0013(3) |
| C(13) | 0,1164(3) | 0,6773(2) | 0,0396(3) |
| C(14) | 0,1380(2) | 0,7977(2) | 0,0394(3) |
| C(15) | 0,2507(3) | 0,8743(2) | -0,0020(3) |
| C(16) | 0,3364(3) | 0,8317(2) | -0,0424(3) |
| C(17) | 0,0528(3) | 0,8477(2) | 0,0923(3) |
| C(18) | -0,0898(3) | 0,7975(2) | 0,0473(3) |
| C(19) | -0,1577(3) | 0,8494(3) | 0,1117(3) |
| C(20) | -0,0879(3) | 0,9494(3) | 0,2184(4) |
| C(21) | 0,0507(3) | 1,0006(3) | 0,2599(4) |
| C(22) | 0,1205(3) | 0,9498(3) | 0,1983(4) |
| C(23) | -0,1774(2) | 0,6935(2) | -0,0688(3) |
| N(24) | -0,1481(2) | 0,6593(2) | -0,1858(2) |
| N(25) | -0,2625(2) | 0,5661(2) | -0,2540(2) |
| N(26) | -0,3573(2) | 0,5423(2) | -0,1882(3) |
| N(27) | -0,3053(2) | 0,6223(2) | -0,0681(3) |
| C(28) | 0,2116(4) | 0,4603(4) | -0,3254(4) |
| C(29) | 0,1072(5) | 0,3772(4) | -0,4633(5) |
| C(30) | -0,0182(4) | 0,2920(5) | -0,4422(5) |
| C(31) | -0,1105(5) | 0,2132(5) | -0,5811(6) |
| O(32) | 0,5713(3) | 0,8456(2) | -0,2336(3) |

**TABLEAU VII**

| **Distances intramoléculaires dans la Forme B** | | |
|---|---|---|
| atome | atome | distance |
| N(1) | C(5) | 1,370(4) |
| N(1) | C(2) | 1,380(3) |
| N(1) | C(10) | 1,468(3) |
| C(2) | N(3) | 1,279(4) |
| C(2) | C(28) | 1,484(4) |
| N(3) | C(4) | 1,471(4) |
| C(4) | C(5) | 1,513(4) |
| C(4) | C(6) | 1,543(4) |
| C(4) | C(9) | 1,549(4) |
| C(5) | O(32) | 1,202(3) |
| C(6) | C(7) | 1,501(6) |
| C(7) | C(8) | 1,485(7) |
| C(8) | C(9) | 1,507(5) |
| C(10) | C(11) | 1,507(3) |
| C(11) | C(12) | 1,384(4) |
| C(11) | C(16) | 1,396(4) |
| C(12) | C(13) | 1,384(4) |
| C(13) | C(14) | 1,390(4) |
| C(14) | C(15) | 1,399(4) |
| C(14) | C(17) | 1,489(4) |
| C(15) | C(16) | 1,379(4) |
| C(17) | C(22) | 1,395(4) |
| C(17) | C(18) | 1,404(4) |
| C(18) | C(19) | 1,394(4) |
| C(18) | C(23) | 1,477(4) |
| C(19) | C(20) | 1,381(4) |
| C(20) | C(21) | 1,364(5) |
| C(21) | C(22) | 1,389(4) |
| C(23) | N(24) | 1,328(3) |
| C(23) | N(27) | 1,354(3) |
| N(24) | N(25) | 1,324(3) |
| N(25) | N(26) | 1,301(3) |
| N(26) | N(27) | 1,319(3) |
| C(28) | C(29) | 1,519(5) |
| C(29) | C(30) | 1,448(6) |
| C(30) | C(31) | 1,473(6) |

Les distances sont en Ångstroms. Les écart types estimés sur la dernière décimale sont entre parenthèses.

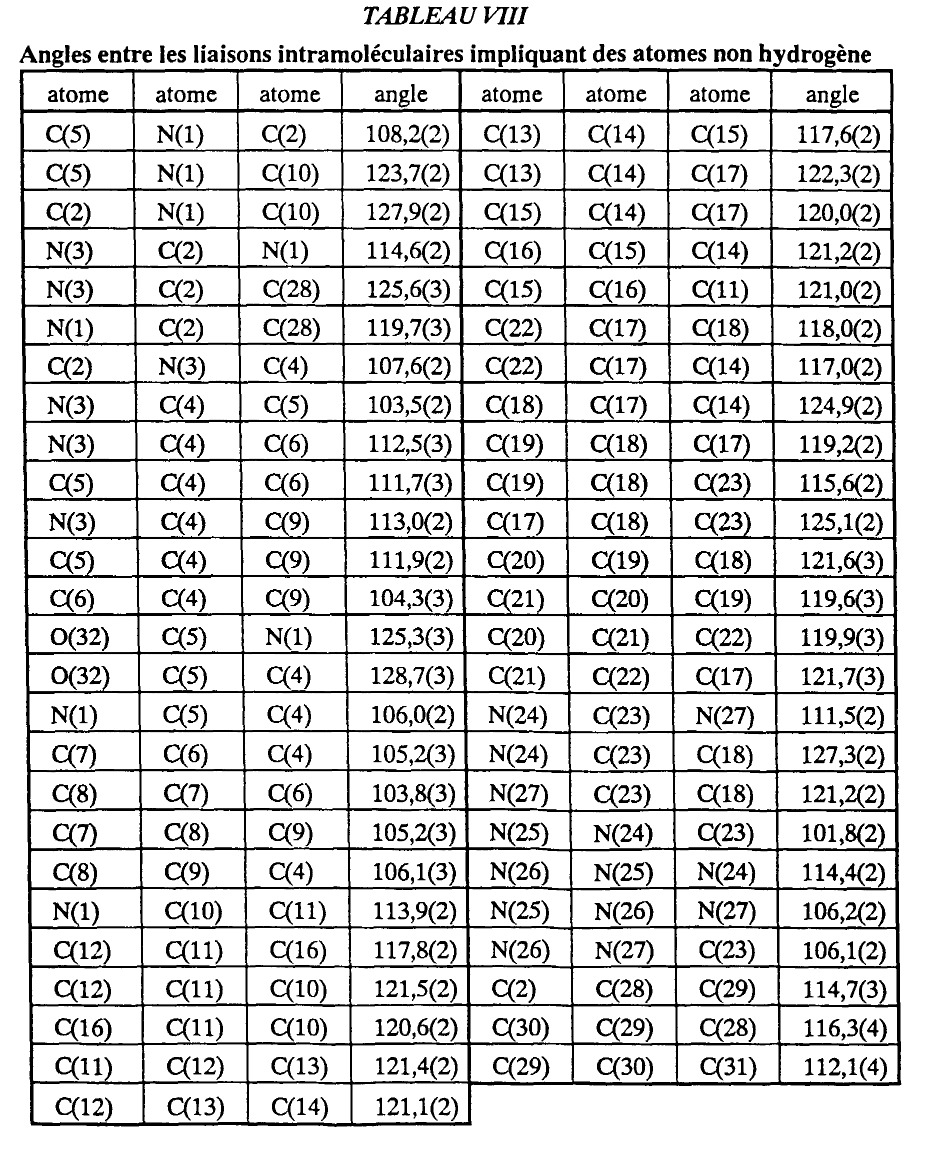

Les angles sont en degrés. Les écart types estimés sur la dernière décimale sont entre parenthèses.

**TABLEAU IX**

| **Angles de conformation et torsion caractéristique** | | | | |
|---|---|---|---|---|
| (1) | (2) | (3) | (4) | angle |
| N(1) | C(10) | C(11) | C(12) | 110,2(3) |
| N(1) | C(10) | C(11) | C(16) | -71,6(3) |
| N(1) | C(2) | C(28) | C(29) | 167,0(4) |
| N(1) | C(2) | N(3) | C(4) | -0,2(3) |
| C(2) | C(28) | C(29) | C(30) | -162,3(5) |
| C(2) | N(1) | C(10) | C(11) | -89,4(3) |
| C(2) | N(1) | C(5) | C(4) | -0,1(3) |
| C(2) | N(3) | C(4) | C(5) | 0,1(3) |
| N(3) | C(2) | C(28) | C(29) | -10,6(6) |
| N(3) | C(4) | C(5) | N(1) | 0,0(3) |
| C(4) | C(6) | C(7) | C(8) | 36,6(5) |
| C(6) | C(4) | C(9) | C(8) | -3,3(4) |
| C(6) | C(7) | C(8) | C(9) | -38,9(5) |
| C(7) | C(8) | C(9) | C(4) | 25,9(5) |
| C(9) | C(4) | C(6) | C(7) | -20,2(4) |
| C(13) | C(14) | C(17) | C(18) | -49,6(4) |
| C(17) | C(18) | C(23) | N(24) | -28,2(4) |
| C(23) | N(24) | N(25) | N(26) | 0,3(3) |
| N(24) | C(23) | N(27) | N(26) | -0,4(3) |
| N(24) | N(25) | N(26) | N(27) | -0,6(3) |
| N(25) | N(26) | N(27) | C(23) | 0,6(3) |
| C(28) | C(29) | C(30) | C(31) | -178,2(5) |

Le signe est positif si, en regardant de l'atome 2 à l'atome 3, par un mouvement dans les sens horaire l'atome 1 se superpose sur l'atome 4.

L'étude cristallographique aux rayons X, notamment les données cristallographiques du Tableau I, les coordonnées atomiques du Tableau VI, la longueur des liaisons du Tableau VII, les angles entre les liaisons du Tableau VIII et les angles de torsion caractéristiques du Tableau IX prouvent la structure (B) proposée et illustrée sur la Figure 3.

L'emplacement de l'hydrogène N(H) sur N(25) est justifié par les faits suivants:
- l'atome H proche de N(25) a été trouvé dans la carte de la Fourier différence;
- une liaison hydrogène intermoléculaire N(25)-H...N(3)[-x,1-y,-1-z] est présente dans le réticule cristallin (Figure 5);
- des quatre angles X-N-Y endocycliques, l'angle N(24)-N(25)-N(26) est le plus large (114,3°), tandis que tous les trois autres sont inférieurs à 110° (Figure 4); selon la théorie V.S.E.P.R., la répulsion de la paire d'électrons solitaires est plus forte que celle dans la liaison N-H.

Par conséquent, la Forme B, à l'état solide, est sous la forme du tautomère 2H-1,2,3,4-tétrazole.

Jusqu'à maintenant, on n'a pas réussi à obtenir un monocristal de la Forme A apte à être analysé aux rayons X. L'examen au microscope a révélé que les cristaux de la nouvelle Forme B sont morphologiquement différents de ceux de la Forme A.

La Forme B de la 2-n.butyl-3-[[2'-(tétrazol-5-yl)biphényl-4-yl]méthyl]-1,3-diazaspiro[4.4]non-1-ène-4-one selon la présente invention est aussi active en tant qu'antagoniste du récepteur de l'angiotensine II et au moins aussi biodisponible que la Forme A connue. Grâce à sa faible électrostaticité par rapport à celle de la Forme A, elle est donc particulièrement adaptée à la fabrication de compositions pharmaceutiques pour le traitement de toutes les maladies dans lesquelles un antagoniste de l'angiotensine II est indiqué, notamment de l'hypertension.

Ainsi, selon un autre de ses aspects, la présente invention a pour objet des compositions pharmaceutiques contenant, comme principe actif, la Forme B de la 2-n.butyl-3-[[2'-(tétrazol-5-yl)biphényl-4-yl]méthyl]-1,3-diazaspiro[4.4]non-1-ène-4-one, caractérisée par le profil de diffraction des rayons X de la poudre illustré dans le Tableau I.

De préférence, la Forme B de la 2-n.butyl-3-[[2'-(tétrazol-5-yl)biphényl-4-yl]-méthyl]-1,3-diazaspiro[4.4]non-1-ène-4-one selon la présente invention est formulée dans des compositions pharmaceutiques par voie orale contenant de 1 à 500 mg de principe actif par unité de dosage, en mélange avec un excipient pharmaceutique.

Lorsqu'on prépare une composition solide sous forme de comprimés, on mélange l'ingrédient actif principal avec un véhicule pharmaceutique, tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Si on souhaite formuler le principe actif pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les exemples suivants illustrent l'invention sans toutefois la limiter. L'abbreviation THF désigne le tétrahydrofurane.

### EXEMPLE 1

(a) Un mélange de 1 kg de 2-n.butyl-3-[(2'-cyanobiphényl-4-yl]méthyl-1,3-diazaspiro[4.4]non-1-ène-4-one, de 713 g de chlorhydrate de triéthylamine, 337 g d'azoture de sodium dans 2 l de 1-méthylpyrrolidin-2-one est chauffé sous agitation pendant 12 heures à une température de 121-123°C, puis est laissé refroidir jusqu'à une température de 40-50°C. Sous agitation, on ajoute une solution aqueuse d'hydroxyde de sodium à 35% et de l'eau et on continue l'agitation pendant 30 minutes à une température de 20-40°C. On arrête l'agitation, on laisse décanter le milieu, on élimine la phase aqueuse et on traite la phase organique avec un mélange eau/toluène 5/2. On agite le milieu pendant 30 minutes à 20-30°C, puis on arrête l'agitation, on laisse décanter, on élimine la phase organique, on lave la phase aqueuse en y ajoutant de l'acétate d'éthyle sous agitation et on recupère la phase aqueuse contenant le sel de sodium de la 2-n.butyl-3-[[2'-(tétrazol-5-yl)biphényl-4-yl]méthyl]-1,3-diazaspiro[4.4]non-1-ène-4-one.
(b) A la solution aqueuse ainsi obtenue, ayant un pH de 9-11, on ajoute lentement de l'acide chlorhydrique à 36% jusqu'à l'obtention d'un pH de 4,7 - 5,3. A la fin de l'addition d'acide chlorhydrique, la précipitation de la 2-n.butyl-3-[[2'-(tétrazol-5-yl)biphényl-4-yl]méthyl]-1,3-diazaspiro[4.4]non-1-ène-4-one est complète. On agite la suspension obtenue pendant une heure à 20-25°C, on recupère le produit, on l'essore à 20-25°C, on le lave à l'eau. Au produit ainsi obtenu, on ajoute un mélange de 500 ml d'isopropanol et 4,5 l d'eau, puis on chauffe le milieu à 50-55°C pendant une heure. On refroidit à 20-25°C et, après une heure à cette température, on essore bien le produit, on lave les cristaux à l'eau, on les sèche à 60°C. Dans une préparation, on a obtenu 949 g de 2-n.butyl-3-[[2'-(tétrazol-5-yl)biphényl-4-yl]méthyl]-1,3-diazaspiro[4.4]non-1-ène-4-one brute, partiellement amorphe, de pureté 98 (rendement: 86%).
(c) Au produit brut ainsi obtenu, on ajoute 16 l d'isopropanol et on chauffe le mélange ainsi obtenu au reflux jusqu'à dissolution complète. On laisse revenir à la température ambiante, puis on filtre les cristaux, on les lave à l'eau et on les sèche. Dans la même préparation, on a obtenu 901,6 g de la Forme A de la 2-n.butyl-3-[[2'-(tétrazol-5-yl)biphényl-4-yl]méthyl]-1,3-diazaspiro[4.4]non-1-ène-4-one identique au produit décrit par C.A. Bernhart et al., J. Med. Chem., 1993, 36, 3371-3380. Les cristaux ainsi obtenus sont très électrostatiques.

### EXEMPLE 2

Dans une autre préparation, en opérant dans les conditions décrites dans l'Exemple 1, étapes (a) et (b), on a obtenu 970 g de 2-n.butyl-3-[[2'-(tétrazol-5-yl)biphényl-4-yl]méthyl]-1,3-diazaspiro[4.4]non-1-ène-4-one brute, partiellement amorphe, de pureté 98% (rendement 88%).
(c) Au produit ainsi obtenu, on ajoute 7,76 l d'éthanol à 95% et 1,94 l d'eau. On porte le mélange au reflux, on chauffe pendant 10 minutes, on interrompt le chauffage et on amorce avec quelques cristaux de la Forme B. On laisse revenir lentement à la température ambiante sous agitation, puis on refroidit à 15°C, on lave avec un mélange éthanol/eau 1/4, on essore et on sèche à 60°C sous pression réduite. Dans cette préparation, on a obtenu 905,8 g (93,4 %) de la Forme B de la 2-n.butyl-3-[[2'-(tétrazol-5-yl)biphényl-4-yl]méthyl]-1,3-diazaspiro[4.4]non-1-ène-4-one ayant un point de fusion de 185,6°C et le profil de diffraction des rayons X consigné dans le Tableau I ci-dessus. Les cristaux de la Forme B sont très peu électrostatiques.

### EXEMPLE 3

On ajoute 80 ml d'éthanol à 95% et 20 ml d'eau à 10 g de 2-n.butyl-3-[[2'-(tétrazol-5-yl)biphényl-4-yl]méthyl]-1,3-diazaspiro[4.4]non-1-ène-4-one Forme A et on chauffe au reflux le mélange ainsi obtenu pendant 10 minutes jusqu'à ce que la solution devient homogène. On laisse revenir à la température ambiante sous agitation, puis on refroidit à 15°C, on lave avec un mélange éthanol/eau 1/4, on essore et on sèche à 60°C. Dans cette préparation, on a obtenu 8,9 g (89%) de la Forme B de la 2-n.butyl-3-[[2'-(tétrazol-5-yl)biphényl-4-yl]méthyl]-1,3-diazaspiro[4.4]non-1-ène-4-one ayant les mêmes caractéristiques que celle de l'exemple 2.

### EXEMPLE 4

A 3 g de la Forme A de la 2-n.butyl-3-[[2'-(tétrazol-5-yl)biphényl-4-yl]méthyl]-1,3-diazaspiro[4.4]non-1-ène-4-one on ajoute 100 ml d'une solution aqueuse à pH 2, acidifiée par l'acide chlorhydrique, puis on agite pendant 24 heures à la température ambiante (20-25°C). On filtre les cristaux et on les sèche sous pression réduite à la température ambiante. On obtient ainsi la Forme B de la 2-n.butyl-3-[[2'-(tétrazol-5-yl)biphényl-4-yl]méthyl]-1,3-diazaspiro[4.4]non-1-ène-4-one, identique au produit de l'Exemple 3.

### EXEMPLE 5

A 3 g de la Forme B de la 2-n.butyl-3-[[2'-(tétrazol-5-yl)biphényl-4-yl]méthyl]-1,3-diazaspiro[4.4]non-1-ène-4-one obtenue selon l'Exemple 3, on ajoute 45 ml d'isopropanol. On chauffe au reflux pendant 10 minutes, on laisse revenir à la température ambiante sous agitation, puis on refroidit à 15°C, on filtre, on essore et on sèche à 60°C sous pression réduite. On obtient ainsi la Forme A de la 2-n.butyl-3-[[2'-(tétrazol-5-yl)biphényl-4-yl]méthyl]-1,3-diazaspiro[4.4]non-1-ène-4-one.

### EXEMPLE 6

A partir de la Forme A de la 2-n.butyl-3-[[2'-(tétrazol-5-yl)biphényl-4-yl]méthyl]-1,3-diazaspiro[4.4]non-1-ène-4-one, on opère exactement comme décrit dans l'Exemple 5, mais après le 10 minutes de reflux, on amorce avec quelques cristaux de la Forme B et on continue l'opération de cristallisation comme illustré dans ledit exemple. On obtient la Forme A de la 2-n.butyl-3-[[2'-(tétrazol-5-yl)biphényl-4-yl]méthyl]-1,3-diazaspiro[4.4]non-1-ène-4-one contenant des traces de la Forme B.

### EXEMPLES 7 -9

A partir de la Forme A de la 2-n.butyl-3-[[2'-(tétrazol-5-yl)biphényl-4-yl]méthyl]-1,3-diazaspiro[4.4]non-1-ène-4-one, suivant le mode opératoire décrit dans l'Exemple 3 mais en faisant varier le rapport éthanol 95%/eau, on obtient le résultat illustré dans le Tableau X.

**TABLEAU X**

| Exemple N° | Ethanol/eau v/v | Amorçage | Résultat |
|---|---|---|---|
| 7 | 12/3 | Forme B | Forme B pure |
| 8 | 5/1 | Forme B | Forme B pure |
| 9 | 6,3/5 | - | Forme B pure |
| Note: les rapports de volumes indiquent les ml de solvants par g de Forme A de départ | | | |

### EXEMPLE 10

A 3 g de la Forme A de la 2-n.butyl-3-[[2'-(tétrazol-5-yl)biphényl-4-yl]méthyl]-1,3-diazaspiro[4.4]non-1-ène-4-one, on ajoute 30 ml d'isopropanol et 15 ml d'eau et on chauffe le milieu au reflux pendant 10 minutes, puis on opère comme décrit dans l'Exemple 3. On obtient ainsi la Forme B de la 2-n.butyl-3-[[2'-(tétrazol-5-yl)biphényl-4-yl]méthyl]-1,3-diazaspiro[4.4]non-1-ène-4-one.

### EXEMPLES 11 - 14

A partir de la Forme A de la 2-n.butyl-3-[[2'-(tétrazol-5-yl)biphényl-4-yl]méthyl]-1,3-diazaspiro[4.4]non-1-ène-4-one, suivant le mode opératoire décrit dans l'Exemple 3 mais en faisant varier le solvant organique et les rapports v/v (ml de solvants par mg de Forme A de départ), on obtient le résultat illustré dans le Tableau XI

**TABLEAU XI**

| Exemple N° | Solvants | v/v | Amorçage | Résultat |
|---|---|---|---|---|
| 11 | THF/eau | 11/1,5 | - | Forme B pure |
| 12 | méthanol/eau | 8/2 | Forme B | Forme B pure |
| 13 | acétone/eau | 18/2 | Forme B | Forme B pure |
| 14 | acétonitrile/eau | 8/2 | Forme B | Forme B pure |

### EXEMPLE 15

(a) A une solution de 3 g de sel de potassium de la 2-n.butyl-3-[[2'-(tétrazol-5-yl)biphényl-4-yl]méthyl]-1,3-diazaspiro[4.4]non-1-ène-4-one dans 30 ml d'eau on ajoute une solution d'acide chlorhydrique jusqu'à un pH de 4,7. On agite le mélange, puis on recueillit la 2-n.butyl-3-[[2'-(tétrazol-5-yl)biphényl-4-yl]méthyl]-1,3-diazaspiro[4.4]non-1-ène-4-one brute ainsi précipitée et on la sèche à 55°C sous pression réduite.
(b) A 1 g du produit ainsi obtenu on ajoute 24 ml d'éthanol à 95% et 6 ml d'eau et on chauffe le mélange 10 minutes au reflux. En opérant ensuite comme décrit dans l'Exemple 3, on obtient la Forme B de la 2-n.butyl-3-[[2'-(tétrazol-5-yl)biphényl-4-yl]méthyl]-1,3-diazaspiro[4.4]non-1-ène-4-one.

### EXEMPLE 16

Composition pharmaceutique pour l'administration orale contenant la Forme B de la la 2-n.butyl-3-[[2'-(tétrazol-5-yl)biphényl-4-yl]méthyl]-1,3-diazaspiro[4.4]non-1-ène-4-one en tant que principe actif.

| | |
|---|---|
| Principe actif | 25,0 mg |
| Lactose (Lactose Extra Fine Crystal HMS®) | 171,0 mg |
| Amidon de mais (Starch®) | 50,0 mg |
| Talc | 25,5 mg |
| Silice anhydre colloïdale (Aerosil 200®) | 0,5 mg |
| Stéarate de magnésium | 1,0 mg |

On mélange préalablement les ingrédients et on effectue un tamissage préalable, puis on melange intimement et on tamise à deux reprises. Enfin, on introduit le mélange ainsi obtenu dans des gélules de taille n° 0 contenant 273 mg de la composition ci-dessus, correspondant à 25 mg de principe actif.

## Revendications

1. Procédé pour la préparation de la 2-n.butyl-3-[[2'-(tétrazol-5-yl)biphényl-4-yl]-méthyl]-1,3-diazaspiro[4.4]non-1-ène-4-one caractérisé en ce que :
(a) on traite la 2-n.butyl-3-[(2'-cyanobiphényl-4-yl)méthyl]-1,3-diazaspiro[4.4]non-1-ène-4-one avec un azoture alcalin et le chlorhydrate de triéthylamine dans un solvant aprotique polaire inerte et on recupére la 2-n.butyl-3-[[2'-(tétrazol-5-yl)biphényl-4-yl]méthyl]-1,3-diazaspiro[4.4]non-1-ène-4-one ainsi obtenue sous forme d'un de ses sels alcalins en solution aqueuse;
(b) on neutralise en milieu aqueux le sel alcalin de la 2-n.butyl-3-[[2'-(tétrazol-5-yl)biphényl-4-yl]méthyl]-1,3-diazaspiro[4.4]non-1-ène-4-one ainsi obtenu jusqu'à un pH de 4,7 à 5,3; et
(c) on cristallise le produit ainsi précipité
- soit dans un solvant contenant moins d'environ 10% en volume d'eau pour isoler la 2-n.butyl-3-[[2'-(tétrazol-5-yl)biphényl-4-yl]méthyl]-1,3-diazaspiro[4.4]non-1-ène-4-one sous sa Forme A, ayant le profil de diffraction des rayons X de la poudre illustré dans le Tableau II ci-après :
**TABLEAU II**
| **d** | **I/I**_{**0**} |
|---|---|
| 18,98 | 100,00 |
| 10,89 | 5,81 |
| 9,49 | 7,43 |
| 8,48 | 6,60 |
| 7,13 | 46,23 |
| 6,68 | 11.25 |
| 6,30 | 7,45 |
| 5,45 | 8,85 |
| 5,22 | 16,82 |
| 5,03 | 11,81 |
| 4,71 | 15,91 |
| 4,58 | 45,40 |
| 4,44 | 26,12 |
| 4,32 | 25,44 |
| 4,22 | 25,86 |
| 4,11 | 21,72 |
| 3,93 | 25,46 |
| 3,85 | 33,89 |
| 3,77 | 27,76 |
| 3,38 | 9,09 |
| 3,33 | 11,75 |
| 3,23 | 13,68 |
| 3,14 | 11,99 |
| 2,80 | 8,97 |
| 2,71 | 9,50 |
- soit dans un solvant miscible à l'eau contenant plus d'environ 10% en volume d'eau pour isoler la 2-n.butyl-3-[[2'-(tétrazol-5-yl)biphényl-4-yl]méthyl]-1,3-diazaspiro[4.4]non-1-ène-4-one sous sa Forme B, ayant le profil de diffraction des rayons X de la poudre illustré dans le Tableau I ci-après :
**TABLEAU I**
| **d** | **I/I**_{**0**} |
|---|---|
| 11,22 | 100,00 |
| 7,90 | 12,02 |
| 7,52 | 13,79 |
| 7,23 | 18,60 |
| 6,27 | 20,14 |
| 6,09 | 6,47 |
| 5,86 | 7,42 |
| 5,60 | 98,76 |
| 5,41 | 19,45 |
| 5,05 | 24,67 |
| 4,97 | 20,36 |
| 4,91 | 12,92 |
| 4,80 | 27,33 |
| 4,61 | 15,90 |
| 4,49 | 14,73 |
| 4,36 | 9,86 |
| 4,17 | 62,84 |
| 4,07 | 15,39 |
| 3,97 | 30,34 |
| 3,88 | 14,32 |
| 3,83 | 13,56 |
| 3,75 | 37,28 |
| 3,53 | 26,48 |
| 3,46 | 12,42 |
| 3,40 | 27,88 |
| 3,27 | 11,03 |
| 3,18 | 10,42 |
| 3,15 | 7,28 |
| 3,12 | 6,11 |
| 3,05 | 15,50 |
| 3,01 | 9,49 |
| 2,81 | 7,11 |
| 2,78 | 9,40 |

2. Procédé selon la revendication 1, caractérisé en ce que dans l'étape (a), on opère avec l'azoture de sodium dans un solvant choisi parmi diméthylformamide et 1-méthylpyrrolidin-2-one à une température entre 110 et 140°C.

3. Procédé selon la revendication 1, caractérisé en ce que dans l'étape (c), on utilise soit l'isopropanol pour isoler la Forme A, soit un mélange éthanol/eau pour isoler la Forme B.

4. Procédé pour la préparation de la Forme B de la 2-n.butyl-3-[[2'-(tétrazol-5-yl)-biphényl-4-yl]méthyl]-1,3-diazaspiro[4.4]non-1-ène-4-one, caractérisé en ce qu'on recristallise la 2-n.butyl-3-[[2'-(tétrazol-5-yl)biphényl-4-yl]méthyl]-1,3-diazaspiro[4.4]non-1-ène-4-one, sous forme de produit brut ou sous la Forme A dans un solvant miscible à l'eau contenant au moins 10% d'eau.

5. Forme B de la 2-n.butyl-3-[[2'-(tétrazol-5-yl)biphényl-4-yl]méthyl]-1,3-diazaspiro[4.4]non-1-ène-4-one ayant le profil de diffraction des rayons X de la poudre illustré dans le Tableau I figurant dans la revendication 1.

6. Forme B de la 2-n.butyl-3-[[2'-(tétrazol-5-yl)biphényl-4-yl]méthyl]-1,3-diazaspiro[4.4]non-1-ène-4-one selon la revendication 5, caractérisée en ce qu'elle possède :
- un point de fusion de 185 - 186°C;
- des absorptions caractéristiques lR à 1537, 1200 et 745 cm⁻¹.

7. Composition pharmaceutique contenant, en tant que principe actif, la Forme B de la 2-n.butyl-3-[[2'-(tétrazol-5-yl)biphényl-4-yl]méthyl]-1,3-diazaspiro[4.4]-non-1-ène-4-one selon les revendications 5 à 6.

## Patentansprüche

1. Verfahren zur Herstellung von 2-n.Butyl-3-[[2'-(tetrazol-5-yl)-biphenyl-4-yl]-methyl]-1,3-diazaspiro[4.4]non-1-en-4-on, dadurch gekennzeichnet, dass
(a) 2-n.Butyl-3-[(2'-cyanobiphenyl-4-yl)-methyl]-1,3-diazaspiro[4.4]non-1-en-4-on mit einem Alkalinitrid und Triethylaminhydrochlorid in einem inerten polaren aprotischen Lösungsmittel behandelt und das so erhaltene 2-n.Butyl-3-[[2'-(tetrazol-5-yl)-biphenyl-4-yl]-methyl]-1,3-diazaspiro[4.4]non-1-en-4-on in Form eines seiner Alkalisalze in wässeriger Lösung gewonnen wird;
(b) das so erhaltene Alkalisalz von 2-n.Butyl-3-[[2'-(tetrazol-5-yl)-biphenyl-4-yl]-methyl]-1,3-diazaspiro[4.4]non-1-en-4-on in wässerigem Medium auf einen pH-Wert von 4,7 bis 5,3 neutralisiert wird; und
(c) das so ausgefällte Produkt kristallisiert wird, u.zw.
- entweder in einem Lösungsmittel, das weniger als etwa 10 Vol.-% Wasser enthält, um 2-n.Butyl-3-[[2'-(tetrazol-5-yl)-biphenyl-4-yl]-methyl]-1,3-diazaspiro[4.4]non-1-en-4-on in seiner A-Form zu isolieren, die das in der nachstehenden Tabelle II veranschaulichte Pulver-Röntgendiffraktionsprofil aufweist:
**TABELLE II**
| **d** | **I/I**_{**0**} |
|---|---|
| 18,98 | 100,00 |
| 10,89 | 5,81 |
| 9,49 | 7,43 |
| 8,48 | 6,60 |
| 7,13 | 46,23 |
| 6,68 | 11.25 |
| 6,30 | 7,45 |
| 5,45 | 8,85 |
| 5,22 | 16,82 |
| 5,03 | 11,81 |
| 4,71 | 15,91 |
| 4,58 | 45,40 |
| 4,44 | 26,12 |
| 4,32 | 25,44 |
| 4,22 | 25,86 |
| 4,11 | 21,72 |
| 3,93 | 25,46 |
| 3,85 | 33,89 |
| 3,77 | 27,76 |
| 3,38 | 9,09 |
| 3,33 | 11,75 |
| 3,23 | 13,68 |
| 3,14 | 11,99 |
| 2,80 | 8,97 |
| 2,71 | 9,50 |
- oder in einem mit Wasser mischbaren Lösungsmittel, das mehr als etwa 10 Vol.-% Wasser enthält, um 2-n.Butyl-3-[[2'-(tetrazol-5-yl)-biphenyl-4-yl]-methyl]-1,3-diazaspiro[4.4]non-1-en-4-on in seiner B-Form zu isolieren, die das in der nachstehenden Tabelle I veranschaulichte Pulver-Röntgendiffraktionsprofil aufweist:
**TABELLE I**
| **d** | **I/I**_{**0**} |
|---|---|
| 11,22 | 100,00 |
| 7,90 | 12,02 |
| 7,52 | 13,79 |
| 7,23 | 18,60 |
| 6,27 | 20,14 |
| 6,09 | 6,47 |
| 5,86 | 7,42 |
| 5,60 | 98,76 |
| 5,41 | 19,45 |
| 5,05 | 24,67 |
| 4,97 | 20,36 |
| 4,91 | 12,92 |
| 4,80 | 27,33 |
| 4,61 | 15,90 |
| 4,49 | 14,73 |
| 4,36 | 9,86 |
| 4,17 | 62,84 |
| 4,07 | 15,39 |
| 3,97 | 30,34 |
| 3,88 | 14,32 |
| 3,83 | 13,56 |
| 3,75 | 37,28 |
| 3,53 | 26,48 |
| 3,46 | 12,42 |
| 3,40 | 27,88 |
| 3,27 | 11,03 |
| 3,18 | 10,42 |
| 3,15 | 7,28 |
| 3,12 | 6,11 |
| 3,05 | 15,50 |
| 3,01 | 9,49 |
| 2,81 | 7,11 |
| 2,78 | 9,40 |

2. Verfahren nach Anspruch 1, dadurch gekennzeichnet, dass in Stufe (a) mit Natriumnitrid in einem Lösungsmittel ausgewählt aus Dimethylformamid und 1-Methylpyrrolidin-2-on bei einer Temperatur zwischen 110 und 140°C gearbeitet wird.

3. Verfahren nach Anspruch 1, dadurch gekennzeichnet, dass in Stufe (c) entweder Isopropanol zum Isolieren der A-Form oder eine Ethanol/Wasser-Mischung zum Isolieren der B-Form verwendet wird.

4. Verfahren zur Herstellung der B-Form von 2-n.Butyl-3-[[2'-(tetrazol-5-yl)-biphenyl-4-yl]-methyl]-1,3-diazaspiro[4.4]non-1-en-4-on, dadurch gekennzeichnet, dass 2-n.Butyl-3-[[2'-(tetrazol-5-yl)-biphenyl-4-yl]-methyl]-1,3-diazaspiro[4.4]non-1-en-4-on in Form des Rohprodukts oder in der A-Form aus einem mit Wasser mischbaren Lösungsmittel, das mindestens 10 % Wasser enthält, umkristallisiert wird.

5. B-Form von 2-n.Butyl-3-[[2'-(tetrazol-5-yl)-biphenyl-4-yl]-methyl]-1,3-diazaspiro[4.4]non-1-en-4-on mit dem in Tabelle I des Anspruchs 1 veranschaulichten Pulver-Röntgendiffraktionsprofil.

6. B-Form von 2-n.Butyl-3-[[2'-(tetrazol-5-yl)-biphenyl-4-yl]-methyl]-1,3-diazaspiro[4.4]non-1-en-4-on nach Anspruch 5, dadurch gekennzeichnet, dass sie folgendes aufweist:
- einen Schmelzpunkt von 185-186°C;
- charakteristische IR-Absorptionen bei 1537, 1200 und 745 cm⁻¹.

7. Pharmazeutische Zusammensetzung, die als Wirkstoff die B-Form von 2-n.Butyl-3-[[2'-(tetrazol-5-yl)-biphenyl-4-yl]-methyl]-1,3-diazaspiro[4.4]non-1-en-4-on nach den Ansprüchen 5 bis 6 enthält.

## Claims

1. Process for the preparation of the 2-n.butyl-1-[[2'-(tetrazol-5-yl)biphenyl-4-yl]-methyl]-1,3-diazaspiro[4.4]non-1-en-4-one characterized in that :
(a) the 2-n.butyl-3-[(2'-cyanobiphenyl-4-yl)methyl]-1,3-diazaspiro-[4.4]non-1-en-4-one is treated with an alkaline azide and triethylamine hydrochloride in an inert polar aprotic solvent and the thus obtained 2-n.butyl-3-[[2'-(tetrazol-5-yl)biphenyl-4-yl]methyl]-1,3-diaza-spiro[4.4] non-1-en-4-one is recovered in form of one of its alkaline salts in aqueous solution:
(b) the alkaline salt of the thus obtained 2-n.butyl-3-[[2'-(tetrazol-5-yl)biphenyl-4-yl]methyl]-1,3-diazaspiro[4.4]non-1-en-4-one is neutralized in an aqueous medium until the pH is of from 4.7 to 5.3; and
(c) the product thus precipitated is crystallized
- either in a solvent containing less than about 10% in volume of water to isolate the 2-n.butyl-3-[[2'-(tetrazol-5-yl)biphenyl-4-yl]methyl]-1,3-diazaspiro[4.4]non-1-en-4-one in its Form A, having the X-ray powder diffraction pattern given in Table II hereinbelow :
**TABLE II**
| **d** | **I/I**_{**0**} |
|---|---|
| 18.98 | 100.00 |
| 10.89 | 5.81 |
| 9.49 | 7.43 |
| 8.48 | 6.60 |
| 7.13 | 46.23 |
| 6.68 | 11.25 |
| 6.30 | 7.45 |
| 5.45 | 8.85 |
| 5.22 | 16.82 |
| 5.03 | 11.81 |
| 4.71 | 15.91 |
| 4.58 | 45.40 |
| 4.44 | 26.12 |
| 4.32 | 25.44 |
| 4.22 | 25.86 |
| 4.11 | 21.72 |
| 3.93 | 25.46 |
| 3.85 | 33.89 |
| 3.77 | 27.76 |
| 3.38 | 9.09 |
| 3.33 | 11.75 |
| 3.23 | 13.68 |
| 3.14 | 11.99 |
| 2.80 | 8.97 |
| 2.71 | 9.50 |
- or in a water-miscible solvent containing more than about 10% in volume of water to isolate the 2-n.butyl-3-[[2'-(tetrazol-5-yl)biphenyl-4-yl]methyl]-1,3-diazaspiro[4.4]non-1-en-4-one in its Form B, having the X-ray powder diffraction pattern given in Table I hereinbelow :
**TABLE I**
| **d** | **I/I**_{**0**} |
|---|---|
| 11.22 | 100.00 |
| 7.90 | 12.02 |
| 7.52 | 13.79 |
| 7.23 | 18.60 |
| 6.27 | 20.14 |
| 6.09 | 6.47 |
| 5.86 | 7.42 |
| 5.60 | 98.76 |
| 5.41 | 19.45 |
| 5.05 | 24.67 |
| 4.97 | 20.36 |
| 4.91 | 12.92 |
| 4.80 | 27.33 |
| 4.61 | 15.90 |
| 4.49 | 14.73 |
| 4.36 | 9.86 |
| 4.17 | 62.84 |
| 4.07 | 15.39 |
| 3.97 | 30.34 |
| 3.88 | 14.32 |
| 3.83 | 13.56 |
| 3.75 | 37.28 |
| 3.53 | 26.48 |
| 3.46 | 12.42 |
| 3.40 | 27.88 |
| 3.27 | 11.03 |
| 3.18 | 10.42 |
| 3.15 | 7.28 |
| 3.12 | 6.11 |
| 3.05 | 15.50 |
| 3.01 | 9.49 |
| 2.81 | 7.11 |
| 2.78 | 9.40 |

2. Process according to claim 1, characterized in that, in step (a), the reaction is carried out with sodium azide in a solvent selected from the group consisting of dimethylformamide and 1-methylpyrrolidin-2-one at a temperature between 110 and 140°C.

3. Process according to claim 1, characterized in that, in step (c), either isopropanol is used to isolate Form A or an ethanol/water mixture to isolate Form B.

4. A process for the preparation of the Form B of the 2-n.butyl-3-[[2'-(tetrazol-5-yl)biphenyl-4-yl]methyl]-1.3-diazaspiro[4.4]non-1-en-4-one characterized in that the 2-n.butyl-3-[[2'-(tetrazol-5-yl)biphenyl-4-yl]methyl]-1.3-diazaspiro[4.4]non-1-en-4-one in a crude product form or in its Form A is recrystallized from a water-miscible solvent containing at least 10% of water.

5. Form B of 2-n.butyl-3-[[2'-(tetrazol-5-yl)biphenyl-4-yl]methyl]-1.3-diazaspiro-[4.4]non-1-en-4-one having the X-ray powder diffraction pattern illustrated in Table I given in claim 1.

6. Form B of 2-n.butyl-3-[[2'-(tetrazol-5-yl)biphenyl-4-yl]methyl]-1.3-diazaspiro-[4.4]non-1-en-4-one according to claim 5, characterized by having :
- a melting point of 185 - 186°C :
- IR-characteristic absorbances at 1537, 1200 and 745 cm⁻¹.

7. A pharmaceutical composition containing the Form B of the 2-n.butyl-3-[[2'-(tetrazol-5-yl)biphenyl-4-yl]methyl]-1,3-diazaspiro[4.4]non-1-en-4-one according to one of claims 5 to 6, as the active ingredient in combination with a pharmaceutical excipient.
